# EUROPEAN PATENT APPLICATION

(11) **EP 4 316 482 A1**
(43) Date of publication of application: **07.02.2024**
(21) Application number: 22306161.5
(22) Date of filing: 01.08.2022
(51) Int. Cl.: A61K 31/198, A61K 31/137, A61K 31/165, A61K 31/4458, A61P 25/00, A61P 25/30

(54) **LEVODOPA FOR PREVENTING ADDICTION**

(71) Applicant: 4P-Pharma, 59000 Lille (FR)
(72) Inventor: VIEUBLED, Margot, 59580 ANICHE (FR); MARTIN, Céline, 59175 TEMPLEMARS (FR); SANCHEZ LOPEZ, Daniel, 59160 LILLE (FR); MINTSA M'OWONE, David Barthelemy, 59000 LILLE (FR); RATTENBACH, Revital, 75004 PARIS (FR)
(74) Representative: Icosa

(57) **Abstract**

The present invention relates to levodopa for preventing the development of addiction and/or dependence to prescribed stimulants in a subject treated with stimulants. The present invention also relates to the use of a composition, pharmaceutical composition, medicament or kit comprising levodopa and a stimulant for treating diseases.

## Description

### FIELD OF INVENTION

The present invention relates to levodopa for preventing the development of addiction and/or dependence to prescribed stimulants in a subject treated with stimulants. The present invention also relates to the use of a composition, pharmaceutical composition, medicament or kit comprising levodopa and a stimulant for treating diseases.

### BACKGROUND OF INVENTION

Drugs containing stimulants are often abused for non-prescription uses, and this has turned into a very severe problem.

Stimulants (also known as psychostimulants or analeptics) are psychoactive drugs which are widely used for inducing temporary improvements in the mental or physical function in patients, and are available on prescription.

This class of drugs includes actives like methylphenidate (Ritalin, Concerta, Metadate or Methylin), amphetamine (Adderall, Vyvanse, Dexedrine), dexmethylphenidate (Focalin), serdexmethylphenidate, methylphenidate derivatives amphetamine, modafinil (Provigil) etc., or their mixtures. The positive effects of these stimulants include, for example, enhanced alertness, wakefulness, productivity and motivation.

Abuse of Ritalin typically is associated with young people, such as, for example, preadolescents, teenagers, or young adults. The increased use of the drug for treatment of Attention Deficit Hyperactivity Disorder or ADHD (a disorder that is prevalent among young people), has resulted in a corresponding increase in abuse.

By way of illustration, the University of Michigan's Monitoring the Future Survey indicates that 4 percent of high school seniors in the United States abused the drug at least once in the past year. Furthermore, survey of students at a public liberal arts college found that over 50% of survey participants knew other students who had used Ritalin for fun, 16% had used it themselves, and nearly 13% reported their own use included snorting the drug. Although less common, Ritalin is abused among adults as well. Overall, the data suggest that ADHD medication misuse and diversion are common health care problems for stimulant medications, with the prevalence believed to be approximately 5% to 10% of high school students and 5% to 35% of college students, depending on the study. The Drug Enforcement Administration has received reports of Ritalin abuse among diverse segments of the population, ranging from healthcare professionals to street addicts.

In 2004, over 8000 methylphenidate ingestions were reported in US poison center data. The most common reasons for intentional exposure were drug abuse and suicide attempts. An overdose manifests in agitation, hallucinations, psychosis, lethargy, seizures, tachycardia, dysrhythmias, hypertension, and hyperthermia. Benzodiazepines may be used as treatment if agitation, dystonia, or convulsions are present.

Thus, in view of the huge burden of stimulants abuse in terms of economic and human resources, there is an urgent need to provide new treatments for preventing addiction to stimulants, such as methylphenidate and amphetamine.

Herein, the Applicants have surprisingly discovered that levodopa, either alone or in combination with carbidopa, can prevent addiction and/or dependence induced by administration of prescribed stimulants.

### SUMMARY

The present invention relates to a composition comprising levodopa for use in preventing the development of addiction and/or dependence to a stimulant in a subject, wherein said subject is treated or is to be treated with the stimulant.

In one embodiment, said composition prevents the development of sensitization induced by the stimulant in said subject.

In one embodiment, the composition further comprises an inhibitor of aromatic L-amino acid decarboxylase, preferably said inhibitor of aromatic L-amino acid decarboxylase is carbidopa.

In one embodiment, the stimulant is selected from the group comprising or consisting of methylphenidate, amphetamine, dexmethylphenidate, serdexmethylphenidate, methylphenidate derivatives amphetamine, modafinil and combinations thereof, preferably the stimulant is methylphenidate or amphetamine. In one embodiment, the stimulant is the combination of methylphenidate and amphetamine.

In one embodiment, the amount of levodopa in the composition is at least about 10 mg, preferably between about 10 mg to about 500 mg, more preferably between about 50 mg to about 100 mg.

In one embodiment, the amount of carbidopa in the composition is at least about 1 mg, preferably between about 1 mg to about 50 mg, more preferably between about 5 mg to about 10 mg.

In one embodiment, said subject is treated or is to be treated with a dose of stimulants of at least about 1 mg per intake, preferably between about 1 mg to about 200 mg, more preferably between about 10 mg to about 100 mg.

In one embodiment, the ratio of stimulant to levodopa is comprised between 30: 100 and 120: 100, or the ratio of stimulant/levodopa/carbidopa is comprised between 30: 100: 10 and 120: 100: 10.

In one embodiment, said subject suffers from or is diagnosed with attention deficit hyperactivity disorder (ADHD) or narcolepsy.

The present invention also relates to a pharmaceutical composition comprising levodopa and at least one pharmaceutically acceptable excipient, for use in preventing the development of addiction and/or dependence to a stimulant in a subject, wherein said subject is treated with the stimulant.

The present invention also relates to a composition comprising levodopa and a stimulant, preferably wherein said stimulant is selected from the group comprising or consisting of methylphenidate, amphetamine, dexmethylphenidate, serdexmethylphenidate, methylphenidate derivatives amphetamine, modafinil and combinations thereof, more preferably wherein said stimulant is methylphenidate or amphetamine.

In one embodiment, the composition further comprises an inhibitor of aromatic L-amino acid decarboxylase, preferably said inhibitor of aromatic L-amino acid decarboxylase is carbidopa.

In one embodiment, the composition comprises a ratio of stimulant to levodopa comprised between 30: 100 and 120: 100, or a ratio of stimulant/levodopa/carbidopa comprised between 30: 100: 10 and 120: 100: 10.

The present invention also relates to a composition as defined hereinabove for use as a medicament.

The present invention also relates to a composition as defined hereinabove for use in treating ADHD or narcolepsy in a subject in need thereof.

### DEFINITIONS

In the present invention, the following terms have the following meanings:

**"About":** preceding a figure means plus or less 10% of the value of said figure.

**"Addiction":** refers to the observable, measurable, and often pathological activity of an organism that portrays its inability to overcome a habit resulting in an insatiable craving for a substance or for performing certain acts.

**"Amphetamine":** refers to a stimulant having the following formula:

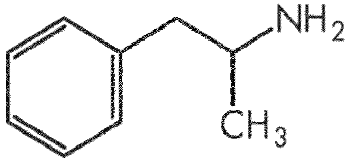

**"Aromatic L-amino acid decarboxylase"** or **"DOPA decarboxylase"** or **"tryptophan decarboxylase"** or **"5-hydroxytryptophan decarboxylase"** or **"AADC"** or "AAD": refers to a lyase enzyme (EC 4.1.1.28), located in region 7p12.2-p12.1. Said enzyme catabolizes several decarboxylation reactions, including the conversion of L-DOPA to dopamine.

**"Carbidopa":** refers to an enzyme that inhibits aromatic L-amino acid decarboxylase. The formula of carbidopa is provided herein below:

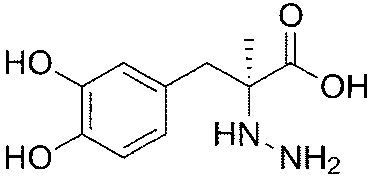

**"Consisting essentially of"**: with reference to a composition, means that i) levodopa, ii) levodopa and an inhibitor of aromatic L-amino acid decarboxylase, iii) levodopa and a stimulant, or iv) levodopa and an inhibitor of aromatic L-amino acid decarboxylase and a stimulant, is/are the only therapeutic agent(s) or agent(s) with a biologic activity within said composition.

"**Dose**": refers to the amount of active agent administered at one time. Preferably, doses are administered to one subject from 4 hours to 1 week apart, such as 24 hours apart. More preferably, said doses are human doses, wherein a human dose is a standard human dose for a man weighing 70 kg. However, it is well known by the skilled artisan in the art that the specific therapeutically effective dose level for any particular patient will depend upon a variety of factors including, for example, the weight, the age, the severity of the disease to be treated, and the tolerance of the treatment.

**"Dependence":** refers to a physical dependence on a substance. Dependence is characterized by the symptoms of tolerance (*i.e.* an increased amount of drugs required to achieve a certain effect) and withdrawal (*i.e.* drug-specific physical and mental symptoms caused by the cessation of drug use).

"**Levodopa**" or "**L-DOPA**": refers to L-3,4-dihydroxyphenylalanine, wherein the formula is provided hereinbelow:

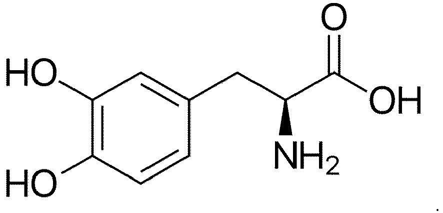

"**Methylphenidate**" or "**MPH**": refers to a stimulant having the following formula:

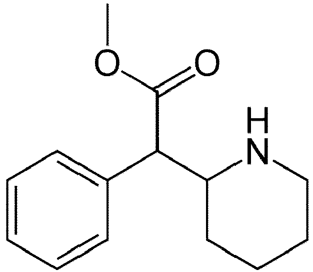

**"Pharmaceutically acceptable excipient":** includes any and all solvents, dispersion media, coatings, antibacterial and antifungal agents, isotonic and absorption delaying agents and the like. Said excipient does not produce an adverse, allergic or other untoward reaction when administered to an animal, preferably a mammal, more preferably a human. For human administration, preparations should meet sterility, pyrogenicity, and general safety and purity standards as required by regulatory offices, such as, for example, FDA Office or EMA.

**"Prevent"** or **"preventing":** refers to any action which makes it possible to prevent at least one symptom associated with a pathological condition, or to prevent the underlying cause of a pathological condition. Said term encompasses full prevention, wherein the subject treated or to be treated with a stimulant, and i) levodopa or ii) levodopa and an inhibitor of aromatic L-amino acid decarboxylase, does not develop addiction and/or dependence to the stimulant. Said term also encompasses partial prevention, wherein the subject treated or to be treated with a stimulant, and i) levodopa or ii) levodopa and an inhibitor of aromatic L-amino acid decarboxylase, develops fewer symptoms or less severe symptoms of addiction and/or dependence than he/she would have with the stimulant only.

**"Sensitization":** refers to the process in which repeated administration of a stimulus results in the progressive amplification of a response.

**"Stimulants":** refers to drugs that tend to increase behavioral alertness, agitation, and/or excitation. As used herein, **"prescribed stimulants"** refers to stimulants prescribed by a health care professional. Examples of stimulants include, without limitation, methylphenidate (Ritalin, Concerta, Metadate or Methylin), amphetamine (Aderall, Vyvanse), dexmethylphenidate, serdexmethylphenidate, methylphenidate derivatives amphetamine, modafinil (Provigil), and their mixtures.

**"Subject"** refers to a mammalian animal, wherein "mammalian animal" refers to a human or a non-human mammalian animal. Preferably, "subject" refers to a human (man or woman).

**"Therapeutically effective amount",** as used herein, refers to the level or amount of the composition, pharmaceutical composition, medicament or kit according to the present invention, that is aimed at (but without causing significant negative or adverse side effects to the subject): (1) delaying or preventing the onset of the targeted condition or disorder; (2) slowing down or stopping the progression, aggravation, or deterioration of one or more symptoms of the targeted condition or disorder; (3) bringing about ameliorations of the symptoms of the targeted condition or disorder; (4) reducing the severity or incidence of the targeted condition or disorder; and/or (5) curing the targeted condition or disorder. A therapeutically effective amount of the composition, pharmaceutical composition, medicament or kit according to the present invention may be administered prior to the onset of the targeted condition or disorder, for a prophylactic or preventive action. Alternatively or additionally, the therapeutically effective amount of the composition, pharmaceutical composition, medicament or kit according to the present invention may be administered after initiation of the targeted condition or disorder, for a therapeutic action.

"**Treating**" or "**treatment**": refers to any action which makes it possible to delay, reduce in severity and/or frequency or suppress at least one symptom associated with a pathological condition, or to slow down or suppress the underlying cause of a pathological condition, or the improvement or remediation of damage. In one embodiment, "treatment" refers to a curative treatment.

### DETAILED DESCRIPTION

In one aspect, the present invention relates to a composition comprising, consisting essentially of or consisting of levodopa for use in preventing the development of addiction and/or dependence to a stimulant in a subject, wherein said subject is treated or is to be treated with the stimulant.

In one embodiment, the stimulant is a prescribed stimulant.

In one embodiment, the stimulant is to be administered concomitantly with the composition as defined hereinabove. In one embodiment, the stimulant is comprised in the composition as defined hereinabove.

In one embodiment, the stimulant is to be administered before the composition as defined hereinabove. In one embodiment, the stimulant is to be administered after the composition as defined hereinabove.

In one embodiment, the stimulant is selected from the group comprising or consisting of methylphenidate, amphetamine, dexmethylphenidate, serdexmethylphenidate, methylphenidate derivatives amphetamine, modafinil and combinations thereof.

In one embodiment, the stimulant is methylphenidate. In one embodiment, the stimulant is amphetamine. In one embodiment, the stimulant is the combination of methylphenidate and amphetamine.

In one embodiment, the dose of stimulant, preferably methylphenidate or amphetamine, is at least about 1 mg per intake. In one embodiment, the dose of stimulant is between about 1 mg to about 200 mg, preferably between about 5 mg to about 150 mg, more preferably between about 10 mg to about 100 mg.

In one embodiment, the dose of stimulant is about 1 mg. In one embodiment, the dose of stimulant is about 2 mg. In one embodiment, the dose of stimulant is about 3 mg. In one embodiment, the dose of stimulant is about 4 mg. In one embodiment, the dose of stimulant is about 5 mg. In one embodiment, the dose of stimulant is about 6 mg. In one embodiment, the dose of stimulant is about 7 mg. In one embodiment, the dose of stimulant is about 8 mg. In one embodiment, the dose of stimulant is about 9 mg. In one embodiment, the dose of stimulant is about 10 mg. In one embodiment, the dose of stimulant is about 11 mg. In one embodiment, the dose of stimulant is about 12 mg. In one embodiment, the dose of stimulant is about 13 mg. In one embodiment, the dose of stimulant is about 14 mg. In one embodiment, the dose of stimulant is about 15 mg. In one embodiment, the dose of stimulant is about 16 mg. In one embodiment, the dose of stimulant is about 17 mg. In one embodiment, the dose of stimulant is about 18 mg. In one embodiment, the dose of stimulant is about 19 mg. In one embodiment, the dose of stimulant is about 20 mg. In one embodiment, the dose of stimulant is about 30 mg. In one embodiment, the dose of stimulant is about 40 mg. In one embodiment, the dose of stimulant is about 60 mg. In one embodiment, the dose of stimulant is about 80 mg. In one embodiment, the dose of stimulant is about 100 mg. In one embodiment, the dose of stimulant is about 120 mg. In one embodiment, the dose of stimulant is about 150 mg. In one embodiment, the dose of stimulant is about 200 mg.

In one embodiment, the dose of stimulant, preferably methylphenidate or amphetamine, is between about 18 mg to about 120 mg, preferably between about 18 mg to about 60 mg.

In one embodiment, the dose of stimulant, preferably methylphenidate or amphetamine, is about 18 mg. In one embodiment, the dose of stimulant, preferably methylphenidate or amphetamine, is about 60 mg. In one embodiment, the dose of stimulant, preferably methylphenidate or amphetamine, is about 120 mg.

The total dose of stimulant may be administered by multiple doses or in a single dose.

In one embodiment, the stimulant is to be administered regularly, such as, from three times per day to one time per month, more preferably from three times per day to one time per two weeks, even more preferably from three times per day to one time per week.

In one embodiment, the stimulant is to be administered one, two or three time(s) per day. In one embodiment, the stimulant is to be administered one time per week.

In one embodiment, doses of the stimulant are to be administered to the subject from 4 hours to 1 week apart, such as 24 hours apart.

In one embodiment, the amount of levodopa in the composition is at least about 10 mg. In one embodiment, the amount of levodopa in the composition is between about 10 mg to about 500 mg, preferably between about 20 mg to about 200 mg, more preferably between about 50 mg to about 100 mg.

In one embodiment, the amount of levodopa in the composition is about 10 mg. In one embodiment, the amount of levodopa in the composition is about 11 mg. In one embodiment, the amount of levodopa in the composition is about 12 mg. In one embodiment, the amount of levodopa in the composition is about 13 mg. In one embodiment, the amount of levodopa in the composition is about 14 mg. In one embodiment, the amount of levodopa in the composition is about 15 mg. In one embodiment, the amount of levodopa in the composition is about 16 mg. In one embodiment, the amount of levodopa in the composition is about 17 mg. In one embodiment, the amount of levodopa in the composition is about 18 mg. In one embodiment, the amount of levodopa in the composition is about 19 mg. In one embodiment, the amount of levodopa in the composition is about 20 mg. In one embodiment, the amount of levodopa in the composition is about 40 mg. In one embodiment, the amount of levodopa in the composition is about 50 mg. In one embodiment, the amount of levodopa in the composition is about 60 mg. In one embodiment, the amount of levodopa in the composition is about 80 mg. In one embodiment, the amount of levodopa in the composition is about 100 mg. In one embodiment, the amount of levodopa in the composition is about 120 mg. In one embodiment, the amount of levodopa in the composition is about 150 mg. In one embodiment, the amount of levodopa in the composition is about 200 mg. In one embodiment, the amount of levodopa in the composition is about 250 mg. In one embodiment, the amount of levodopa in the composition is about 300 mg. In one embodiment, the amount of levodopa in the composition is about 350 mg. In one embodiment, the amount of levodopa in the composition is about 400 mg. In one embodiment, the amount of levodopa in the composition is about 450 mg. In one embodiment, the amount of levodopa in the composition is about 500 mg.

In one embodiment, the amount of levodopa in the composition is about 50 mg. In one embodiment, the amount of levodopa in the composition is about 100 mg.

In one embodiment, the ratio of stimulant, preferably methylphenidate or amphetamine, to levodopa is from 1: 100 to 10 000: 100, preferably from 20: 100 to 150: 100, more preferably from 30: 100 to 120: 100.

In one embodiment, the ratio of stimulant to levodopa is 1: 100. In one embodiment, the ratio of stimulant to levodopa is 2: 100. In one embodiment, the ratio of stimulant to levodopa is 4: 100. In one embodiment, the ratio of stimulant to levodopa is 6: 100. In one embodiment, the ratio of stimulant to levodopa is 8: 100. In one embodiment, the ratio of stimulant to levodopa is 10: 100. In one embodiment, the ratio of stimulant to levodopa is 18: 100. In one embodiment, the ratio of stimulant to levodopa is 20: 100. In one embodiment, the ratio of stimulant to levodopa is 30: 100. In one embodiment, the ratio of stimulant to levodopa is 40: 100. In one embodiment, the ratio of stimulant to levodopa is 60: 100. In one embodiment, the ratio of stimulant to levodopa is 80: 100. In one embodiment, the ratio of stimulant to levodopa is 1: 1. In one embodiment, the ratio of stimulant to levodopa is 120: 100. In one embodiment, the ratio of stimulant to levodopa is 140: 100. In one embodiment, the ratio of stimulant to levodopa is 150: 100. In one embodiment, the ratio of stimulant to levodopa is 160: 100. In one embodiment, the ratio of stimulant to levodopa is 180: 100. In one embodiment, the ratio of stimulant to levodopa is 200: 100. In one embodiment, the ratio of stimulant to levodopa is 220: 100. In one embodiment, the ratio of stimulant to levodopa is 240: 100. In one embodiment, the ratio of stimulant to levodopa is 260: 100. In one embodiment, the ratio of stimulant to levodopa is 280: 100. In one embodiment, the ratio of stimulant to levodopa is 300: 100. In one embodiment, the ratio of stimulant to levodopa is 400: 100. In one embodiment, the ratio of stimulant to levodopa is 500: 100. In one embodiment, the ratio of stimulant to levodopa is 1 000: 100. In one embodiment, the ratio of stimulant to levodopa is 10 000: 100.

In one embodiment, the ratio of stimulant, preferably methylphenidate or amphetamine, to levodopa is from 18: 100 to 120: 100, preferably from 18: 100 to 60: 100. In one embodiment, the ratio of stimulant, preferably methylphenidate or amphetamine, to levodopa is 18: 100. In one embodiment, the ratio of stimulant, preferably methylphenidate or amphetamine, to levodopa is 36: 100. In one embodiment, the ratio of stimulant, preferably methylphenidate or amphetamine, to levodopa is 60: 100. In one embodiment, the ratio of stimulant, preferably methylphenidate or amphetamine, to levodopa is 120: 100.

In one embodiment, the composition as defined hereinabove further comprises an inhibitor of aromatic L-amino acid decarboxylase.

Examples of inhibitors of aromatic L-amino acid decarboxylase include, without limitation, benserazide (CAS number 14919-77-8), carbidopa (CAS number 8860-95-9), the S-enantiomer of methyldopa (CAS number 555-30-6), alpha-difluoromethyl-DOPA (DFMD) (CAS number 160401-53-6), 3',4',5,7-tetrahydroxy-8-methoxyisoflavone (CAS number 58262-89-8), epigallocatechin gallate (EGCG) (CAS number 989-51-5), and epigallocatechin (EGC) (CAS number 970-74-1).

In one embodiment, the inhibitor of aromatic L-amino acid decarboxylase is carbidopa.

In one embodiment, the amount of an inhibitor of aromatic L-amino acid decarboxylase in the composition is at least 1 mg. In one embodiment, the amount of an inhibitor of aromatic L-amino acid decarboxylase in the composition is between about 1 mg to about 100 mg, preferably of about 1 mg to about 50 mg, more preferably between about 5 mg to about 10 mg.

In one embodiment, the amount of an inhibitor of aromatic L-amino acid decarboxylase in the composition is about 1 mg. In one embodiment, the amount of an inhibitor of aromatic L-amino acid decarboxylase in the composition is about 2 mg. In one embodiment, the amount of an inhibitor of aromatic L-amino acid decarboxylase in the composition is about 3 mg. In one embodiment, the amount of an inhibitor of aromatic L-amino acid decarboxylase in the composition is about 4 mg. In one embodiment, the amount of an inhibitor of aromatic L-amino acid decarboxylase in the composition is about 5 mg. In one embodiment, the amount of levodopa in the composition is about 6 mg. In one embodiment, the amount of an inhibitor of aromatic L-amino acid decarboxylase in the composition is about 7 mg. In one embodiment, the amount of an inhibitor of aromatic L-amino acid decarboxylase in the composition is about 8 mg. In one embodiment, the amount of an inhibitor of aromatic L-amino acid decarboxylase in the composition is about 9 mg. In one embodiment, the amount of an inhibitor of aromatic L-amino acid decarboxylase in the composition is about 10 mg. In one embodiment, the amount of an inhibitor of aromatic L-amino acid decarboxylase in the composition is about 11 mg. In one embodiment, the amount of an inhibitor of aromatic L-amino acid decarboxylase in the composition is about 12 mg. In one embodiment, the amount of an inhibitor of aromatic L-amino acid decarboxylase in the composition is about 13 mg. In one embodiment, the amount of an inhibitor of aromatic L-amino acid decarboxylase in the composition is about 14 mg. In one embodiment, the amount of an inhibitor of aromatic L-amino acid decarboxylase in the composition is about 15 mg. In one embodiment, the amount of an inhibitor of aromatic L-amino acid decarboxylase in the composition is about 16 mg. In one embodiment, the amount of an inhibitor of aromatic L-amino acid decarboxylase in the composition is about 17 mg. In one embodiment, the amount of an inhibitor of aromatic L-amino acid decarboxylase in the composition is about 18 mg. In one embodiment, the amount of an inhibitor of aromatic L-amino acid decarboxylase in the composition is about 19 mg. In one embodiment, the amount of an inhibitor of aromatic L-amino acid decarboxylase in the composition is about 20 mg. In one embodiment, the amount of an inhibitor of aromatic L-amino acid decarboxylase in the composition is about 25 mg. In one embodiment, the amount of an inhibitor of aromatic L-amino acid decarboxylase in the composition is about 40 mg. In one embodiment, the amount of an inhibitor of aromatic L-amino acid decarboxylase in the composition is about 50 mg. In one embodiment, the amount of an inhibitor of aromatic L-amino acid decarboxylase in the composition is about 60 mg. In one embodiment, the amount of an inhibitor of aromatic L-amino acid decarboxylase in the composition is about 80 mg. In one embodiment, the amount of an inhibitor of aromatic L-amino acid decarboxylase in the composition is about 100 mg.

In one embodiment, the amount of an inhibitor of aromatic L-amino acid decarboxylase in the composition, preferably carbidopa, is about 5 mg. In one embodiment, the amount of an inhibitor of aromatic L-amino acid decarboxylase in the composition, preferably carbidopa, is about 10 mg.

In one embodiment, the ratio of levodopa to an inhibitor of aromatic L-amino acid decarboxylase is from 1: 1 to 100: 1, more preferably from 5: 1 to 50: 1, more preferably of 10: 1.

In one embodiment, the ratio of stimulant/levodopa/inhibitor of aromatic L-amino acid decarboxylase, preferably methylphenidate/levodopa/carbidopa or amphetamine/levodopa/carbidopa, is from 1: 100: 10 to 10 000: 100: 10, preferably from 50: 100: 10 to 150: 100: 10, more preferably from 30: 100: 10 to 120: 100: 10.

In one embodiment, the ratio of stimulant/levodopa/inhibitor of aromatic L-amino acid decarboxylase is 1 100 10. In one embodiment, the ratio of stimulant/levodopa/inhibitor of aromatic L-amino acid decarboxylase is 2: 100: 10. In one embodiment, the ratio of stimulant/levodopa/inhibitor of aromatic L-amino acid decarboxylase is 4: 100: 10. In one embodiment, the ratio of stimulant/levodopa/inhibitor of aromatic L-amino acid decarboxylase is 6: 100: 10. In one embodiment, the ratio of stimulant/levodopa/inhibitor of aromatic L-amino acid decarboxylase is 8: 100: 10. In one embodiment, the ratio of stimulant/levodopa/inhibitor of aromatic L-amino acid decarboxylase is 10: 100: 10. In one embodiment, the ratio of stimulant/levodopa/inhibitor of aromatic L-amino acid decarboxylase is 18: 100: 10. In one embodiment, the ratio of stimulant/levodopa/inhibitor of aromatic L-amino acid decarboxylase is 20: 100: 10. In one embodiment, the ratio of stimulant/levodopa/inhibitor of aromatic L-amino acid decarboxylase is 30: 100: 10. In one embodiment, the ratio of stimulant/levodopa/inhibitor of aromatic L-amino acid decarboxylase is 35: 100: 10. In one embodiment, the ratio of stimulant/levodopa/inhibitor of aromatic L-amino acid decarboxylase is 40: 100: 10. In one embodiment, the ratio of stimulant/levodopa/inhibitor of aromatic L-amino acid decarboxylase is 60: 100: 10. In one embodiment, the ratio of stimulant/levodopa/inhibitor of aromatic L-amino acid decarboxylase is 80: 100: 10. In one embodiment, the ratio of stimulant/levodopa/inhibitor of aromatic L-amino acid decarboxylase is 1 : 1: 10. In one embodiment, the ratio of stimulant/levodopa/inhibitor of aromatic L-amino acid decarboxylase is 120: 100: 10. In one embodiment, the ratio of stimulant/levodopa/inhibitor of aromatic L-amino acid decarboxylase is 140: 100: 10. In one embodiment, the ratio of stimulant/levodopa/inhibitor of aromatic L-amino acid decarboxylase is 150: 100: 10. In one embodiment, the ratio of stimulant/levodopa/inhibitor of aromatic L-amino acid decarboxylase is 160: 100: 10. In one embodiment, the ratio of stimulant to levodopa is 180: 100. In one embodiment, the ratio of stimulant/levodopa/inhibitor of aromatic L-amino acid decarboxylase is 200: 100: 10. In one embodiment, the ratio of stimulant/levodopa/inhibitor of aromatic L-amino acid decarboxylase is 220: 100: 10. In one embodiment, the ratio of stimulant/levodopa/inhibitor of aromatic L-amino acid decarboxylase is 240: 100: 10. In one embodiment, the ratio of stimulant/levodopa/inhibitor of aromatic L-amino acid decarboxylase is 260: 100: 10. In one embodiment, the ratio of stimulant/levodopa/inhibitor of aromatic L-amino acid decarboxylase is 280: 100: 10. In one embodiment, the ratio of stimulant/levodopa/inhibitor of aromatic L-amino acid decarboxylase is 300: 100: 10. In one embodiment, the ratio of stimulant/levodopa/inhibitor of aromatic L-amino acid decarboxylase is 400: 100: 10. In one embodiment, the ratio of stimulant/levodopa/inhibitor of aromatic L-amino acid decarboxylase is 500: 100: 10. In one embodiment, the ratio of stimulant/levodopa/inhibitor of aromatic L-amino acid decarboxylase is 1 000: 100: 10. In one embodiment, the ratio of stimulant/levodopa/inhibitor of aromatic L-amino acid decarboxylase is 10 000: 100: 10.

In one embodiment, the ratio of stimulant/levodopa/inhibitor of aromatic L-amino acid decarboxylase, preferably methylphenidate/levodopa/carbidopa or amphetamine/levodopa/carbidopa, is from 18: 100: 10 to 120: 100: 10, preferably from 18: 100: 10 to 60: 100: 10. In one embodiment, the ratio of stimulant/levodopa/inhibitor of aromatic L-amino acid decarboxylase, preferably methylphenidate/levodopa/carbidopa or amphetamine/levodopa/carbidopa, is 18: 100: 10. In one embodiment, the ratio of stimulant/levodopa/inhibitor of aromatic L-amino acid decarboxylase, preferably methylphenidate/levodopa/carbidopa or amphetamine/levodopa/carbidopa, is 36: 100: 10. In one embodiment, the ratio of stimulant/levodopa/inhibitor of aromatic L-amino acid decarboxylase, preferably methylphenidate/levodopa/carbidopa or amphetamine/levodopa/carbidopa, is 60: 100: 10. In one embodiment, the ratio of stimulant/levodopa/inhibitor of aromatic L-amino acid decarboxylase, preferably methylphenidate/levodopa/carbidopa or amphetamine/levodopa/carbidopa, is 120: 100: 10.

The present invention also relates to a pharmaceutical composition comprising, consisting essentially of or consisting of levodopa and at least one pharmaceutically acceptable excipient, for use in preventing the development of addiction and/or dependence to a stimulant in a subject, wherein said subject is treated or is to be treated with the stimulant.

In one embodiment, the pharmaceutical composition for use according to the present invention further comprises an inhibitor of aromatic L-amino acid decarboxylase, such as, carbidopa.

In one embodiment, the pharmaceutical composition for use according to the present invention further comprises the stimulant.

In one embodiment, the pharmaceutical composition comprises from 0.1% to 99% by weight, preferably from 1% to 50% by weight, more preferably from 5% to 25% by weight, even more preferably about 20% by weight, of levodopa, relative to the total weight of said pharmaceutical composition.

In one embodiment, the pharmaceutical composition comprises from 0.1% to 99% by weight, preferably from 1% to 50% by weight, more preferably from 5% to 25% by weight, even more preferably about 20% by weight, of levodopa and an inhibitor of aromatic L-amino acid decarboxylase, relative to the total weight of said pharmaceutical composition.

In one embodiment, the pharmaceutical composition comprises from 0.1% to 99% by weight, preferably from 1% to 50% by weight, more preferably from 5% to 25% by weight, even more preferably about 20% by weight, of levodopa and an inhibitor of aromatic L-amino acid decarboxylase and a stimulant, relative to the total weight of said pharmaceutical composition.

Examples of pharmaceutically acceptable excipients that may be used in the compositions of the present invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances (for example sodium carboxymethylcellulose), polyethylene glycol, polyacrylates, waxes, polyethylene- polyoxypropylene- block polymers, polyethylene glycol and wool fat.

In one embodiment, the pharmaceutical composition according to the present invention comprises vehicles which are pharmaceutically acceptable for a formulation capable of being injected to a subject. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The present invention further relates a medicament comprising, consisting essentially of or consisting of levodopa for use in preventing the development of addiction and/or dependence to a stimulant in a subject, wherein said subject is treated or is to be treated with the stimulant.

In one embodiment, the medicament for use according to the present invention further comprises an inhibitor of aromatic L-amino acid decarboxylase as defined hereinabove. In one embodiment, the inhibitor of aromatic L-amino acid decarboxylase is carbidopa.

In one embodiment, the medicament for use according to the present invention further comprises the stimulant.

The present invention further relates to the use of the composition or pharmaceutical composition as defined hereinabove for the manufacture of a medicament for preventing the development of addiction and/or dependence to a stimulant in a subject, wherein said subject is treated or is to be treated with the stimulant.

The present invention further relates to a method of preventing the development of addiction and/or dependence to a stimulant in a subject, wherein said subject is treated or is to be treated with the stimulant, comprising administering to said subject an effective amount of the composition, pharmaceutical composition, or medicament according to the present invention.

For use in administration to a subject, the composition, pharmaceutical composition or medicament will be formulated for administration to the subject.

The composition, pharmaceutical composition, or medicament may be administered by enteral or parenteral route of administration.

The enteral route may be selected from the group consisting of buccal route (including perlingual route and sublingual route), oral route and rectal route.

In one embodiment, the composition, pharmaceutical composition, or medicament according to the invention to the invention is to be administered by oral route.

For oral administration, the composition, pharmaceutical composition or medicament, may be formulated into conventional oral dosage forms such as tablets, gels, capsules, powders, granules, patches and liquid preparations such as syrups, elixirs, and concentrated drops. Nontoxic solid carriers or diluents may be used which include, for example, pharmaceutical grades of mannitol, lactose, starch, magnesium stearate, sodium saccharine, talcum, cellulose, glucose, sucrose, magnesium, carbonate, and the like. For compressed tablets, binders, which are agents which impart cohesive qualities to powdered materials, are also necessary. For example, starch, gelatine, sugars such as lactose or dextrose, and natural or synthetic gums can be used as binders. Disintegrants are also necessary in the tablets to facilitate break-up of the tablet. Disintegrants include starches, clays, celluloses, algins, gums and crosslinked polymers. Moreover, lubricants and glidants are also included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants.

In one embodiment, the composition, pharmaceutical composition or medicament as defined hereinabove is under an oral dosage form.

The parenteral includes any route that is not enteral. The parenteral route may be selected from the group consisting of epicutaneous route, transdermal route, intradermal route, subcutaneous route, nasal route, intramuscular route, intraocular route, intravitreal route, and intravitreal route.

In one embodiment, the composition, pharmaceutical composition, or medicament according to the present invention is to be administered by subcutaneous route.

Examples of forms adapted for injection include, but are not limited to, solutions, such as, for example, sterile aqueous solutions, gels, dispersions, emulsions, suspensions, solid forms suitable for using to prepare solutions or suspensions upon the addition of a liquid prior to use, such as, for example, powder, liposomal forms and the like.

Sterile injectable forms of the compositions, pharmaceutical compositions or medicaments of this invention may be aqueous or an oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

In one embodiment, the composition, pharmaceutical composition, or medicament according to the invention is to be administered regularly, such as, from three times per day to one time per month, more preferably from three times per day to one time per two weeks, even more preferably from three times per day to one time per week.

In one embodiment, the composition, pharmaceutical composition, or medicament according to the invention is to be administered one, two or three time(s) per day. In one embodiment, the composition, pharmaceutical composition, or medicament is to be administered one time per week.

In one embodiment, doses of the composition, pharmaceutical composition, or medicament according to the invention are to be administered to the subject from 4 hours to 1 week apart, such as 24 hours apart.

In one embodiment, the composition, pharmaceutical composition, or medicament according to the present invention is to be administered each time the stimulant is administered.

In one embodiment, the subject is treated or is to be treated with a stimulant as defined hereinabove.

In one embodiment, the subject is treated or is to be treated regularly with the stimulant and/or the composition, pharmaceutical composition, or medicament according to the present invention, such as, from three times per day to one time per month, more preferably from three times per day to one time per two weeks, even more preferably from three times per day to one time per week.

In one embodiment, the subject is treated or is to be treated one, two or three time(s) per day with the stimulant and/or the composition, pharmaceutical composition, or medicament according to the present invention. In one embodiment, the subject is treated or is to be treated one time per week with the stimulant and/or the composition, pharmaceutical composition, or medicament according to the present invention.

In one embodiment, the subject is treated or is to be treated at the same time with the composition, pharmaceutical composition or medicament according to the present invention and the stimulant, meaning that the subject receives the composition, pharmaceutical composition or medicament according to the present invention concomitantly with the stimulant.

In one embodiment, the subject is treated or is to be treated with the composition, pharmaceutical composition or medicament according to the present invention before or after receiving the simulant.

The stimulant as defined hereinabove may be administered in a subject to treat or prevent a disease or a disorder. Thus, in one embodiment, the subject suffers from or is diagnosed with a disease or a condition that is treated or is to be treated with a stimulant as defined hereinabove.

In one embodiment, the subject suffers from or is diagnosed with attention deficit hyperactivity disorder (ADHD).

As used herein, ADHD is a behavior disorder originating in childhood in which the essential features are signs of developmentally inappropriate inattention, impulsivity, and hyperactivity. Although most individuals have symptoms of both inattention and hyperactivity-impulsivity, one or the other pattern may be predominant.

Diagnostic criteria for ADHD according to DSM-5 are described in Table 1 hereinbelow:

**Table 1**

| Symptoms and/or behaviors that have persisted ≥ 6 months in ≥ 2 settings (e.g., school, home, church). Symptoms have negatively impacted academic, social, and/or occupational functioning. In patients aged < 17 years, ≥ 6 symptoms are necessary; in those aged ≥ 17 years, ≥ 5 symptoms are necessary | |
|---|---|
| Inattentive Type Diagnosis Criteria | • Displays poor listening skills |
| | • Loses and/or misplaces items needed to complete activities or tasks |
| | • Sidetracked by external or unimportant stimuli |
| | • Forgets daily activities |
| | • Diminished attention span |
| | • Lacks ability to complete schoolwork and other assignments or to follow instructions |
| | • Avoids or is disinclined to begin homework or activities requiring concentration |
| | • Fails to focus on details and/or makes thoughtless mistakes in schoolwork or assignments |
| Hyperactive/ Impulsive Type Diagnosis Criteria | Hyperactive Symptoms: |
| | • Squirms when seated or fidgets with feet/hands |
| | • Marked restlessness that is difficult to control |
| | • Appears to be driven by "a motor" or is often "on the go" |
| | • Lacks ability to play and engage in leisure activities in a quiet manner |
| | • Incapable of staying seated in class |
| | • Overly talkative |
| | Impulsive Symptoms: |
| | • Difficulty waiting turn |
| | • Interrupts or intrudes into conversations and activities of others |
| | • Impulsively blurts out answers before questions completed |
| Additional Requirements for Diagnosis | • Symptoms present prior to age 12 years |
| | • Symptoms not better accounted for by a different psychiatric disorder (e.g., mood disorder, anxiety disorder) and do not occur exclusively during a psychotic disorder (e.g., schizophrenia) |
| | • Symptoms not exclusively a manifestation of oppositional behavior |
| Classification | Combined Tvpe: |
| | • Patient meets both inattentive and hyperactive/impulsive criteria for the past 6 months |
| | Predominantly Inattentive Tvpe: |
| | • Patient meets inattentive criterion, but not hyperactive/impulse criterion, for the past 6 months |
| | Predominantly Hyperactive/Impulsive Type: |
| | • Patient meets hyperactive/impulse criterion, but not inattentive criterion, for the past 6 months |
| | Symptoms may be classified as mild, moderate, or severe based on symptom severity |

In one embodiment, the subject presents with at least one symptom of ADHD Examples of symptoms of ADHD are provided in Table 1 hereinabove.

In one embodiment, the subject suffers from or is diagnosed with narcolepsy.

As used herein, narcolepsy is a condition characterized by recurrent episodes of daytime somnolence and lapses in consciousness (microsomnias) that may be associated with automatic behaviors and amnesia.

Methods for diagnosing narcolepsy are well-known by the skilled artisan in the art, and include, without limitation, investigating sleep history by questions, such as, for example, the Epworth Sleepiness Scale, performing sleep records, polysomnography, and multiple sleep latency test.

In one embodiment, the subject present with at least one symptom of narcolepsy.

Examples of symptoms of narcolepsy include, without limitation, excessive daytime sleepiness, sudden loss of muscle tone, sleep paralysis, changes in rapid eye movement (REM) sleep and hallucinations.

In one embodiment, the subject is a child, *i.e.* with an age below 18 years. In one embodiment, the subject has an age below 18, 17, 16, 15, 14, 13, 12, 11, 10, 9, 8, 7, 6, 5, 4, 3, 2 or 1 year(s).

In one embodiment, the subject is an adult, *i.e.* with an age equal or above 18 years. In one embodiment, the subject has an age above 18, 20, 25, 30, 35, 40, 45, 50, 55, 60, 65, 70, 75 or 80 years.

In one embodiment, the subject does not have history of addiction before receiving the composition, pharmaceutical composition or medicament as defined hereinabove.

In one embodiment, the subject is not addict and/or dependent to the stimulant before receiving the composition, pharmaceutical composition or medicament as defined hereinabove.

In one embodiment, the composition, pharmaceutical composition or medicament of the present invention prevents the development of addiction to a stimulant in a subject, wherein said subject is treated or is to be treated with the stimulant. In one embodiment, said addiction is induced by the regular or chronic administration of the stimulant.

In one embodiment, the composition, pharmaceutical composition or medicament of the present invention prevents the development of at least one symptom of addiction to a stimulant in a subject, wherein said subject is treated or is to be treated with the stimulant.

Examples of symptoms of addiction include, without limitation, an inability to stop using, use and abuse of substances continue despite health problems.

In one embodiment, the composition, pharmaceutical composition or medicament of the present invention prevents the development of dependence to a stimulant in a subject, wherein said subject is treated or is to be treated with the stimulant.

In one embodiment, the composition, pharmaceutical composition or medicament of the present invention prevents the development of at least one symptom of dependence to a stimulant in a subject, wherein said subject is treated or is to be treated with the stimulant.

Examples of symptoms of dependence include, without limitation, tolerance and withdrawal.

In one embodiment, the composition, pharmaceutical composition or medicament of the present invention prevents the development of sensitization, preferably behavioral sensitization, induced by the administration of the stimulant, preferably methylphenidate or amphetamine, in the subject. In one embodiment, said sensitization is induced by the regular or chronic administration of the stimulant.

In one embodiment, the composition, pharmaceutical composition or medicament of the present invention prevents the hyperactivity induced by the administration of the stimulant, preferably methylphenidate or amphetamine, in the subject. In one embodiment, said hyperactivity is induced by the acute or chronic administration of the stimulant.

In another aspect, the present invention relates to a composition, pharmaceutical composition, or medicament comprising, consisting essentially of or consisting of levodopa and a stimulant.

The present invention also relates to a kit of parts comprising, in a first part, levodopa and, in a second part, a stimulant.

In one embodiment, the stimulant is as defined hereinabove.

In one embodiment, the amount of stimulant in the composition, pharmaceutical composition, medicament or kit as defined hereinabove is at least about 1 mg. In one embodiment, the amount of stimulant in the composition, pharmaceutical composition, medicament or kit as defined hereinabove is between about 1 mg to about 200 mg, preferably between about 5 to about 150 mg, more preferably between about 10 mg to about 100 mg.

In one embodiment, the amount of stimulant in the composition, pharmaceutical composition, medicament or kit is about 1 mg. In one embodiment, the amount of stimulant in the composition, pharmaceutical composition, medicament or kit is about 2 mg. In one embodiment, the amount of stimulant in the composition, pharmaceutical composition, medicament or kit is about 3 mg. In one embodiment, the amount of stimulant in the composition, pharmaceutical composition, medicament or kit is about 4 mg. In one embodiment, the amount of stimulant in the composition, pharmaceutical composition, medicament or kit is about 5 mg. In one embodiment, the amount of stimulant in the composition, pharmaceutical composition, medicament or kit is about 6 mg. In one embodiment, the amount of stimulant in the composition, pharmaceutical composition, medicament or kit is about 7 mg. In one embodiment, the amount of stimulant in the composition, pharmaceutical composition, medicament or kit is about 8 mg. In one embodiment the amount of stimulant in the composition, pharmaceutical composition, medicament or kit is about 9 mg. In one embodiment, the amount of stimulant in the composition, pharmaceutical composition, medicament or kit is about 10 mg. In one embodiment, the amount of stimulant in the composition, pharmaceutical composition, medicament or kit is about 11 mg. In one embodiment, the amount of stimulant in the composition, pharmaceutical composition, medicament or kit is about 12 mg. In one embodiment, the amount of stimulant in the composition, pharmaceutical composition, medicament or kit is about 13 mg. In one embodiment, the amount of stimulant in the composition, pharmaceutical composition, medicament or kit is about 14 mg. In one embodiment, the amount of stimulant in the composition, pharmaceutical composition, medicament or kit is about 15 mg. In one embodiment, the amount of stimulant in the composition, pharmaceutical composition, medicament or kit is about 16 mg. In one embodiment, the amount of stimulant in the composition, pharmaceutical composition, medicament or kit is about 17 mg. In one embodiment, the amount of stimulant in the composition, pharmaceutical composition, medicament or kit is about 18 mg. In one embodiment, the amount of stimulant in the composition, pharmaceutical composition, medicament or kit is about 19 mg. In one embodiment, the amount of stimulant in the composition, pharmaceutical composition, medicament or kit is about 20 mg. In one embodiment, the amount of stimulant in the composition, pharmaceutical composition, medicament or kit is about 30 mg. In one embodiment, the amount of stimulant in the composition, pharmaceutical composition, medicament or kit is about 40 mg. In one embodiment, the amount of stimulant in the composition, pharmaceutical composition, medicament or kit is about 60 mg. In one embodiment, the amount of stimulant in the composition, pharmaceutical composition, medicament or kit is about 80 mg. In one embodiment, the amount of stimulant in the composition, pharmaceutical composition, medicament or kit is about 100 mg. In one embodiment, the amount of stimulant in the composition, pharmaceutical composition, medicament or kit is about 120 mg. In one embodiment, the amount of stimulant in the composition, pharmaceutical composition, medicament or kit is about 150 mg. In one embodiment, the amount of stimulant in the composition, pharmaceutical composition, medicament or kit is about 200 mg.

In one embodiment, the amount of stimulant, preferably methylphenidate or amphetamine, in the composition, pharmaceutical composition, medicament or kit is between about 18 mg to about 120 mg, preferably between about 18 mg to about 60 mg. In one embodiment, the amount of stimulant, preferably methylphenidate or amphetamine, in the composition, pharmaceutical composition, medicament or kit is about 18 mg. In one embodiment, the amount of stimulant, preferably methylphenidate or amphetamine, in the composition, pharmaceutical composition, medicament or kit is about 60 mg. In one embodiment, the amount of stimulant, preferably methylphenidate or amphetamine, in the composition, pharmaceutical composition, medicament or kit is about 120 mg.

In one embodiment, the amount of levodopa in the composition, pharmaceutical composition, medicament or kit is as defined hereinabove. In one embodiment, the amount of levodopa in the composition, pharmaceutical composition, medicament or kit is at least about 10 mg. In one embodiment, the amount of levodopa in the composition is between about 10 mg to about 500 mg, preferably between about 20 to about 200 mg, more preferably about 50 mg to about 100 mg.

In one embodiment, the amount of levodopa in the composition, pharmaceutical composition, medicament or kit is about 50 mg. In one embodiment, the amount of levodopa in the composition, pharmaceutical composition, medicament or kit is about 100 mg.

In one embodiment, the ratio of stimulant to levodopa is as defined hereinabove. In one embodiment, the ratio of stimulant preferably methylphenidate or amphetamine, to levodopa is from 1: 100 to 10 000: 100, preferably from 20: 100 to 150: 100, more preferably from 30: 100 to 120: 100.

In one embodiment, the ratio of stimulant, preferably methylphenidate or amphetamine, to levodopa is from 18: 100 to 120: 100, preferably from 18: 100 to 60: 100. In one embodiment, the ratio of stimulant, preferably methylphenidate or amphetamine, to levodopa is 18: 100. In one embodiment, the ratio of stimulant preferably methylphenidate or amphetamine, to levodopa is 36: 100. In one embodiment, the ratio of stimulant preferably methylphenidate or amphetamine, to levodopa is 60: 100. In one embodiment, the ratio of stimulant, preferably methylphenidate or amphetamine, to levodopa is 120: 100.

In one embodiment, the composition, pharmaceutical composition or medicament comprising, consisting essentially of or consisting of levodopa and a stimulant as defined hereinabove, further comprises an inhibitor of aromatic L-amino acid decarboxylase.

In one embodiment, the kit of parts as defined hereinabove further comprises, in a third part, an inhibitor of aromatic L-amino acid decarboxylase.

Examples of inhibitors of aromatic L-amino acid decarboxylase are provided hereinabove. In one embodiment, the inhibitor of aromatic L-amino acid decarboxylase is carbidopa.

In one embodiment, the amount of an inhibitor of aromatic L-amino acid decarboxylase in the composition, pharmaceutical composition, medicament or kit is as defined hereinabove. In one embodiment, the amount of an inhibitor of aromatic L-amino acid decarboxylase in the composition, pharmaceutical composition, medicament or kit is at least 1 mg. In one embodiment, the amount of an inhibitor of aromatic L-amino acid decarboxylase in the composition, pharmaceutical composition, medicament or kit is between about 1 mg to about 100 mg, preferably between about 1 mg to about 50 mg, more preferably between about 5 mg to about 10 mg.

In one embodiment, the amount of an inhibitor of aromatic L-amino acid decarboxylase in the composition, preferably carbidopa, is about 5 mg. In one embodiment, the amount of an inhibitor of aromatic L-amino acid decarboxylase in the composition, preferably carbidopa, is about 10 mg.

In one embodiment, the ratio of levodopa to inhibitor of aromatic L-amino acid decarboxylase is from 1: 1 to 100: 1, more preferably from 5: 1 to 50: 1, more preferably of 10: 1.

In one embodiment, the ratio of stimulant/levodopa/inhibitor of aromatic L-amino acid decarboxylase is as defined hereinabove. In one embodiment, the ratio of stimulant/levodopa/inhibitor of aromatic L-amino acid decarboxylase, preferably methylphenidate/levodopa/carbidopa or amphetamine/levodopa/carbidopa, is from 1: 100: 10 to 10 000: 100: 10, preferably from 50: 100: 10 to 150: 100: 10, more preferably from 30: 100: 10 to 120: 100: 10.

In one embodiment, the ratio of stimulant/levodopa/inhibitor of aromatic L-amino acid decarboxylase, preferably methylphenidate/levodopa/carbidopa or amphetamine/levodopa/carbidopa, is from 18: 100: 10 to 120: 100: 10, preferably from 18: 100: 10 to 60: 100: 10. In one embodiment, the ratio of stimulant/levodopa/inhibitor of aromatic L-amino acid decarboxylase, preferably methylphenidate/levodopa/carbidopa or amphetamine/levodopa/carbidopa, is 18: 100: 10. In one embodiment, the ratio of stimulant/levodopa/inhibitor of aromatic L-amino acid decarboxylase, preferably methylphenidate/levodopa/carbidopa or amphetamine/levodopa/carbidopa, is 36: 100: 10. In one embodiment, the ratio of stimulant/levodopa/inhibitor of aromatic L-amino acid decarboxylase, preferably methylphenidate/levodopa/carbidopa or amphetamine/levodopa/carbidopa, is 60: 100: 10. In one embodiment, the ratio of stimulant/levodopa/inhibitor of aromatic L-amino acid decarboxylase, preferably methylphenidate/levodopa/carbidopa or amphetamine/levodopa/carbidopa, is 120: 100: 10.

In one embodiment, the composition, pharmaceutical composition, medicament or kit as defined hereinabove is for use as a medicament.

In one embodiment, the composition, pharmaceutical composition, medicament or kit as defined hereinabove is for use in treating a disease or condition that is to be treated with the stimulant of said composition, pharmaceutical composition, medicament or kit.

In one embodiment, the composition, pharmaceutical composition, medicament or kit as defined hereinabove is for use in treating ADHD in a subject in need thereof.

The present invention further relates to a method of treating ADHD in a subject in need thereof, comprising administering to said subject an effective amount of the composition, pharmaceutical composition, medicament or components of the kit as defined above.

The present invention further relates to the use of the composition, pharmaceutical composition, or kit as defined hereinabove for the manufacture of a medicament for treating ADHD in a subject in need thereof.

In one embodiment, the subject is as defined hereinabove.

In one embodiment, the composition, pharmaceutical composition, medicament or kit as defined hereinabove is for use in treating narcolepsy in a subject in need thereof.

The present invention further relates to a method of treating narcolepsy in a subject in need thereof, comprising administering to said subject an effective amount of the composition, pharmaceutical composition, medicament or components of the kit as defined above.

The present invention further relates to the use of the composition, pharmaceutical composition, or kit as defined hereinabove for the manufacture of a medicament for treating narcolepsy in a subject in need thereof.

In one embodiment, the subject is as defined hereinabove.

In one embodiment, the formulations, routes of administrations and dosage regimens of the composition, pharmaceutical composition, medicament are as defined hereinabove.

In one embodiment, the pharmaceutical composition as defined hereinabove further comprises at least one acceptable excipient. Examples of acceptable excipients are provided hereinabove.

In one embodiment, the composition, pharmaceutical composition or medicament as defined hereinabove is to be administered by enteral route, such as oral route. For oral administration, the composition, pharmaceutical composition or medicament as defined hereinabove may be formulated into conventional oral dosage forms such as tablets, gels, capsules, powders, granules, patches and liquid preparations such as syrups, elixirs, and concentrated drops.

In one embodiment, the composition, pharmaceutical composition or medicament as defined hereinabove is to be administered by parenteral route, such as by subcutaneous route.

In one embodiment, the components of the kit of parts are to be administered by different routes of administration. In one embodiment, the components of the kit of parts are to be administered by the same route of administration.

In one embodiment, the components of the kits of parts are to be administered by enteral route, such as, by oral route. In one embodiment, the components of the kits of parts are to be administered by parenteral route, such as, by subcutaneous route.

In one embodiment, the components of the kit of parts are to be administered concomitantly. In one embodiment, the components of the kits of parts are to be administered sequentially.

In one embodiment, the levodopa comprised in the composition, pharmaceutical composition, medicament or kit as defined hereinabove prevents the development of at least one secondary effect induced by the administration, preferably the regular administration, of a stimulant as defined hereinabove.

One of the secondary effects induced by the administration, preferably the regular administration, of a stimulant as defined hereinabove is the development of addiction and/or dependence to the stimulant.

Thus, in one embodiment, the levodopa comprised in the composition, pharmaceutical composition, medicament or kit as defined hereinabove prevents the development of addiction and/or dependence induced by the stimulant comprised in said composition, pharmaceutical composition, medicament or kit.

In one embodiment, the levodopa comprised in the composition, pharmaceutical composition, medicament or kit as defined hereinabove prevents the development of at least one symptom of addiction and/or dependence induced by the stimulant comprised in said composition, pharmaceutical composition, medicament or kit.

Examples of symptoms of addiction and dependence are provided hereinabove.

In one embodiment, the levodopa comprised in the composition, pharmaceutical composition, medicament or kit prevents the development of sensitization, preferably behavioral sensitization, induced by the stimulant comprised in said composition, pharmaceutical composition, medicament or kit.

In one embodiment, the levodopa comprised in the composition, pharmaceutical composition, medicament or kit prevents the hyperactivity induced by the stimulant comprised in said composition, pharmaceutical composition, medicament or kit.

### BRIEF DESCRIPTION OF THE DRAWINGS

**Figure 1** is a histogram showing the effect of levodopa on horizontal activity in mice treated with methylphenidate (10 mg/kg). Mean ± SEM; n=6 per group. ^{∗∗}p<0.01, ^{∗∗∗}p<0.001 compared to methylphenidate group.
**Figure 2** is a histogram showing the effect of levodopa on vertical activity in mice treated with methylphenidate (10 mg/kg). Mean ± SEM; n=6 per group. ^{∗}p<0.05, ^{∗∗}p<0.01 compared to methylphenidate group.
**Figure 3** is a histogram showing the effect of levodopa/carbidopa on horizontal activity in mice treated with methylphenidate (20 mg/kg). Mean ± SEM; n=6 per group. ^{∗∗} p<0.01, ^{∗∗∗} p<0.001 compared to methylphenidate group.
**Figure 4** is a histogram showing the effect of levodopa/carbidopa on vertical activity in mice treated with methylphenidate (20 mg/kg). Mean ± SEM; n=6 per group. ^{∗}p<0.05, ^{∗∗}p<0.01 compared to methylphenidate group.
**Figure 5** is a graph showing the effect of levodopa/carbidopa on distance travelled during conditioning drug sessions in mice treated with methylphenidate (3 mg/kg). Mean ± SEM; n= 9 per group.
**Figure 6** is a graph showing the effect of levodopa/carbidopa in methylphenidate (10 mg/kg) treated female and male mice on locomotor activity. Mean ± SEM; n=15-18 per group. ^{∗∗}p<0.01, ^{∗∗∗}p<0.001 compared to MPH + levodopa/carbidopa group.
**Figure 7** is a graph showing the effect of levodopa/carbidopa in methylphenidate (10 mg/kg) treated female and male mice on horizontal activity. Mean ± SEM; n=15-18 per group. ^{∗}p<0,05, ^{∗∗}p<0.01, ^{∗∗∗}p<0.001, compared to MPH + levodopa/carbidopa group.
**Figure 8** is a graph showing the effect of chronic methylphenidate administration on locomotor activity before treatment administration in female and male mice. Mean ± SEM; n=15-18 per group. ^{∗}p<0,05 compared to MPH + levodopa/carbidopa group.

### EXAMPLES

The present invention is further illustrated by the following examples.

### Example 1: Effect of levodopa (100mg) on methylphenidate-induced hyperactivity in C57B1/6J mice

### Materials and Methods

### Animals

Species / Strain: Mice / C57B1/6J
Gender / Number / Age: Male / 24 / 8 weeks at study initiation
Source: Janvier Labs, France

### Treatments

The Test items and vehicle were administered via intraperitoneal injections according to Table 2.

**Table 2 - Study design**

| Group No. | n = | Animal ID | Treatment | Dose Level (mg/kg) | Corresponding human dose (mg) | ROA | Dose volume | Conc. (mg/ml) |
|---|---|---|---|---|---|---|---|---|
| 1M | 6 | 1,2,9,16, 23,24 | Vehicle | - | - | IP | 10 ml/kg | - |
| 2M | 6 | 3,4,10,1 5,17,18 | Methylphenidate | 10 | 60 | | | 1 |
| 3M | 6 | 5,6,11,1 4,19,20 | Levodopa | 17 | 100 | | | 1,7 |
| 4M | 6 | 7,8,12,1 3,21,22 | Methylphenidate + Levodopa | 10/17 | 60/100 | | | 1/1,7 |

### Observation of mice activity protocol

Mice were subj ected to a behavioral test to evaluate their activity 15 minutes after treatment administration (5 minutes session). Mice were acclimated to the surrounding for 15 minutes before the beginning of the test. At the appropriate time after treatment administration, mice (1 per cage) were placed in the middle of a polyethylene cage measuring 17 × 32 × 14 cm without stainless steel top grill (no access to food or drinking water), without bedding and with a line drawn at the bottom of the cage. Activity was then monitored during 5 minutes session at 15 minutes after treatment administration, measuring different parameters:
- Horizontal activity: the number of times the mice crossed the line drawn at the bottom of the cage during the session was recorded to assess horizontal activity levels.
- Vertical activity: the number of rearing or climbing was recorded to assess vertical activity levels.

The cage was cleaned with water between each animal.

### Results

During this study, observation of mice activity was performed 15 minutes following test items administration recording mice horizontal and vertical activity levels as a marker of hyperactivity.

Horizontal activity levels, corresponding to the number of times the animals crossed the middle line during the 5 minutes session, were measured to compare the groups.

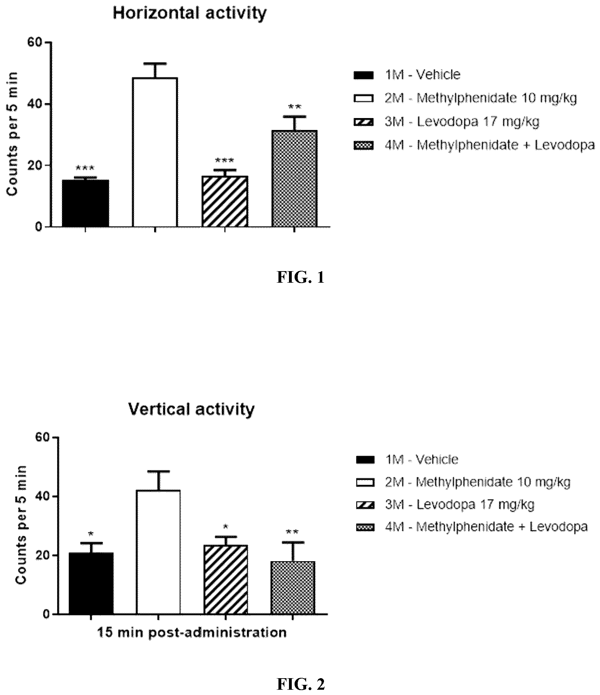

As shown in Figure 1, methylphenidate administration at 10 mg/kg induced a significant increase in mice horizontal activity (49±11 counts) compared to vehicle group (15±2 counts). No significant difference was observed between vehicle group and levodopa treated group (17±4 counts). Levodopa, when administered in combination with methylphenidate 10 mg/kg, significantly reduced mice horizontal activity (32±11 counts) compared to methylphenidate treated group.

Vertical activity corresponds to the number of rearing or climbing during the 5 minutes session.

As shown in Figure 2, methylphenidate treatment at 10 mg/kg induced a significant increase in mice vertical activity (42±16 counts) compared to vehicle-treated one (21±8 counts). No significant difference was observed between vehicle group and levodopa treated group (24±7 counts). Levodopa, when administered in combination with methylphenidate 10 mg/kg, significantly reduces mice vertical activity (18±15 counts) compared to methylphenidate treated group.

In conclusion, it was demonstrated that levodopa, the precursor of dopamine, reduced methylphenidate-induced hyperactivity at a relevant clinical dose (100mg human dose, corresponding to restless leg syndrome dose).

### Example 2: Evaluation of levodopa/carbidopa (100/10mg) on methylphenidate induced hyperactivity in C57B1/6J mice

### Materials and Methods

### Animals

Species / Strain: Mice / C57B1/6J
Gender / Number / Age: Male / 24 / 9 weeks at study initiation
Source: Janvier Labs, France

### Treatments

The Test items and vehicle were administered via intraperitoneal injections according to Table 3.

**Table 3 - Study design**

| Group No. | n = | Animal ID | Treatment | Dose Level (mg/kg) | Correspond ing human dose (mg) | ROA/d ose volume | Conc. (mg/ml) |
|---|---|---|---|---|---|---|---|
| 1M | 6 | 1, 2, 7, 8, 13, 14 | Vehicle | - | - | IP, 10 ml/kg | - |
| 2M | 6 | 3, 4, 9, 10, 15, 16 | Methylphenidate | 20 | 120 | | 2 |
| 3M | 6 | 5, 6, 11, 12, 17, 18 | Levodopa (Carbidopa) + Methylphenidate | 17(1,7)+ 20 | 100 (10) + 120 | | 1.7(0.17) +2 |

### Observation of mice activity protocol

Mice were subj ected to a behavioral test to evaluate their activity 15 minutes after treatment administration (5 minutes session). Mice were acclimated to the surrounding for 15 minutes before the beginning of the test. At the appropriate time after treatment administration, mice (1 per cage) were placed in the middle of a polyethylene cage measuring 17 × 32 × 14 cm without stainless steel top grill (no access to food or drinking water), without bedding and with a line drawn at the bottom of the cage. Activity was then monitored during 5 minutes session at 15 minutes after treatment administration, measuring different parameters:
- Horizontal activity: the number of times the mice crossed the line drawn at the bottom of the cage during the session was recorded to assess horizontal activity levels.
- Vertical activity: the number of rearing or climbing was recorded to assess vertical activity levels.

The cage was cleaned with water between each animal.

### Results

During this study, observation of mice activity was performed 15 minutes following test items administration recording mice horizontal and vertical activity levels as a marker of hyperactivity.

Horizontal activity levels, corresponding to the number of times the animals crossed the middle line during the 5 minutes session, were measured to compare the groups.

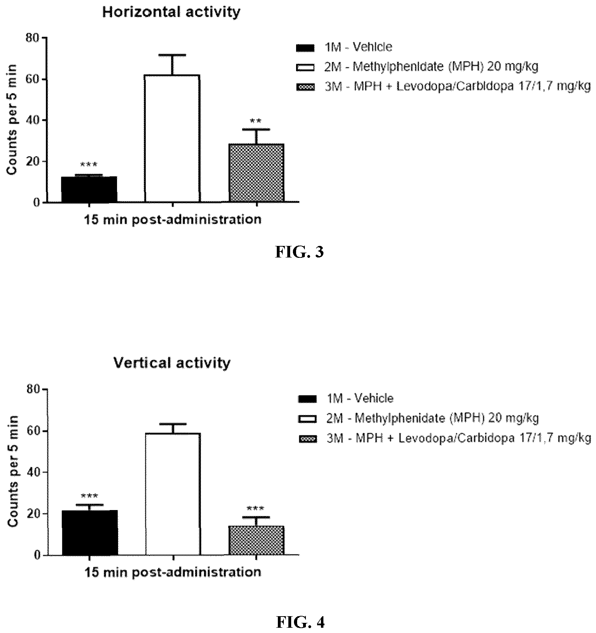

As shown in Figure 3, methylphenidate administration at 20 mg/kg induced a significant increase in mice horizontal activity (62±24 counts) compared to vehicle group (13±2 counts). Levodopa/carbidopa, when administered in combination with methylphenidate 20 mg/kg, significantly reduced mice horizontal activity (29±17 counts) compared to methylphenidate treated group.

Vertical activity corresponds to the number of rearing or climbing during the 5 minutes session.

As shown in Figure 4, methylphenidate treatment at 20 mg/kg induced a significant increase in mice vertical activity (59±10 counts) compared to vehicle-treated one (22±6 counts). Levodopa/carbidopa, when administered in combination with methylphenidate 20 mg/kg, significantly reduce mice vertical activity (14±10 counts) compared to methylphenidate treated group. Mice vertical activity levels in levodopa/carbidopa + methylphenidate-treated group were found similar to vehicle-treated mice.

In conclusion, it was demonstrated that levodopa/carbidopa (100mg/10mg human dose) reduced methylphenidate-induced hyperactivity. Moreover, our data obtained so far demonstrated that a fixed dose levodopa of 100 mg is efficient towards two increasing doses of methylphenidate (60 or 120 mg human dose).

### Example 3: Effect of levodopa/carbidopa on hyperactivity and behavioral sensitization induced by methylphenidate

### Materials and Methods

### Animals

Species / Strain: Mice / C57B1/6J
Gender / Number / Age: Male / 36 / 6-8 weeks at study initiation
Source: Janvier Labs, France

### Treatments

The Test items and vehicle were administered via intraperitoneal injections according to Table 4.

**Table 4 - Study design**

| Group No. | n= | Treatment | Dose Level (mg/kg) | Corresponding human dose (mg) | ROA/dose volume | Conc. (mg/ml) |
|---|---|---|---|---|---|---|
| 1M | 9 | Vehicle | - | - | IP, 10 ml/kg | - |
| 2M | 9 | Methylphenidate | 3 | 18 | | 0,3 |
| 3M | 9 | Levodopa (Carbidopa) + Methylphenidate | 8,5(0,85) +3 | 50(5)+ 18 | | 0,85 (0,085) +3 |
| 4M | 9 | Levodopa (Carbidopa) + Methylphenidate | 17(1,7) +3 | 100 (10) + 18 | | 1.7 (0.17) +0,3 |

### Observation of mice activity protocol

Mice were subj ected to a behavioral test to evaluate their activity 30 minutes after treatment administration. Mice received an administration of vehicle and were placed in the apparatus (20 cm wide, 40 cm large, 40 cm high) for the habituation session (30 minutes). After, 4 drug sessions were performed on days 2, 4, 8 and 10 or on days 3, 5, 9 and 11. Mice were injected with one of the tested drugs (or vehicle) immediately before being placed in the box. Mice activity was then monitored during 30 minutes session after treatment administration, measuring: distance travelled in the compartment in the four sessions.

### Results

During this study, observation of mice activity was performed following test items administration recording mice activity levels as a marker of hyperactivity.

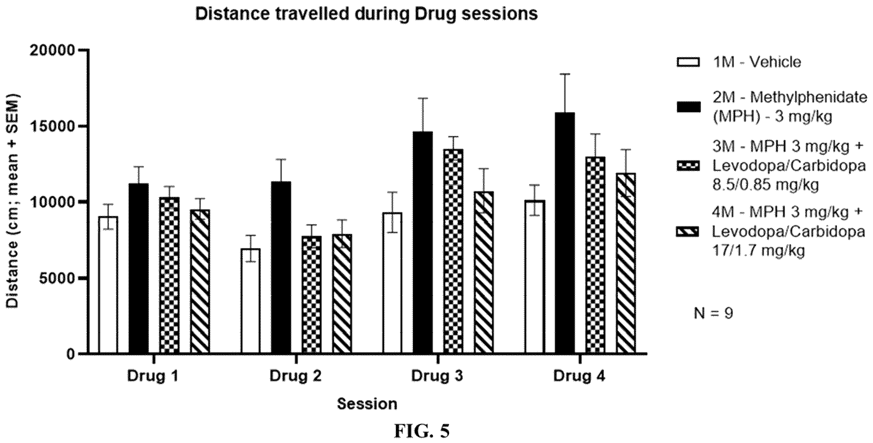

As shown in Figure 5, methylphenidate increased the distance travelled during drug sessions compared to vehicle-treated group and this increase amplified at second, third and fourth sessions. Furthermore, levodopa/carbidopa decreased the increase of activity induced by methylphenidate at the second, third and fourth drug session with dose-dependent effect.

In conclusion, methylphenidate at the dose of 3 mg/kg (18 mg human dose) increased the distance travelled during drug sessions and this increase amplified at the second, third and fourth sessions. Furthermore, levodopa/carbidopa decrease the increased of activity induced by methylphenidate during drug sessions with a dose-dependent effect.

This result shows a decrease by levodopa/carbidopa of hyperactivity and sensitization induced by methylphenidate and therefore suggest a decrease of dependency induced by methylphenidate.

### Example 4: Effect of levodopa/carbidopa on methylphenidate addiction evaluated by a behavioral sensitization test in C57B1/6J mice

### Materials and Methods

### Animals

Species / Strain: Mice / C57B1/6J
Gender / Number / Age: Male / 30 / 9 weeks at study initiation and Female / 35 / 9 weeks at study initiation
Source: Janvier Labs, France

### Treatments

The Test items and vehicle were administered via intraperitoneal injections according to Table 5.

**Table 5 - Study design**

| Group No. | n= | Animal ID | Treatment | Dose Level (mg/kg) | Corresponding human dose (mg) | ROA | Dose volume |
|---|---|---|---|---|---|---|---|
| IF | 9 | 1, 10, 12, 25, 30, 36, 49, 52, 54 | Vehicle | - | - | IP | 10 ml/kg |
| 1M | 7 | 16, 17, 20, 45, 46, 48, 65 | Vehicle | - | - | | |
| 2F | 9 | 7, 9, 11, 27, 31, 33, 50, 53, 56 | Methylphenidate | 10 | 60 | | |
| 2M | 7 | 13, 18, 23, 39, 40, 42, 63 | Methylphenidate | 10 | 60 | | |
| 3F | 9 | 3, 4, 6, 26, 28, 35, 55, 57, 59 | Levodopa/ Carbidopa | 17/1.7 | 100/10 | | |
| 3M | 9 | 14, 19, 21, 37, 41, 43, 62, 64, 66 | Levodopa/ Carbidopa | 17/1.7 | 100/10 | | |
| 4F | 8 | 2, 5, 8, 29, 32, 34, 58, 60 | Methylphenidate + Levodopa/ Carbidopa | 10/17/ 1.7 | 60/100/10 | | |
| 4M | 7 | 15, 22, 24, 38, 44, 47, 61 | Methylphenidate + Levodopa/ Carbidopa | 10/17/ 1.7 | 60/100/10 | | |

### Observation of mice activity protocol

Mice were subjected to a behavioral test to evaluate their activity after treatment or vehicle administration. Mice (1 per cage) were placed in the center of a polyethylene cage without stainless steel top grill (no access to food or drinking water), without bedding and with a line drawn at the bottom of the cage. First, mice were acclimated to the surrounding and video-recorded for 10 minutes before the beginning of the test to analyse the baseline motor activity (total distance travelled). Second, mice were injected with test item or vehicle and acclimated for another 5 minutes and activity was again monitored and video recorded during a 20 minutes session. The 10 minutes video containing basal activity and the 20 minutes video after treatment administration were analysed using the ezTracker-master software. Horizontal activity was also measured. It represents the number of times the mice crossed the line drawn at the bottom of the cage. Animals are submitted to 6 sessions (3 sessions/week on Monday, Wednesday and Friday). During session 1, all mice receive an administration of vehicle. During sessions 2-5, groups 1F/1M receive an injection of vehicle; groups 2F/2M receive methylphenidate injection, groups 3F/3M receive levodopa/carbidopa injection and groups 4F/4M receive levodopa/carbidopa and methylphenidate injections. And during session 6, all mice receive an administration of methylphenidate.

### Results

Chronic administration of methylphenidate results in the initiation and intensification of biochemical and behavioral manifestations that lead to dependence on the drug and behavioral sensitization. Behavioral sensitization is characterized by the progressive and persistent increase in response to a psychostimulant repeated exposure.

During this study, observation of mice activity was performed 5 minutes following test items administration recording mice horizontal and locomotor activity levels as a marker of hyperactivity.

### Effects of levodopa/carbidopa on development and expression of behavioral sensitization induced by methylphenidate.

Behavioral sensitization can be separated into two phases: development (or initiation) and expression. Development is the immediate neural events that induce locomotor sensitization, and expression is the long-term consequences of these initial events. So, development of sensitization corresponds at experimental day 3, 5, 8 and 10.

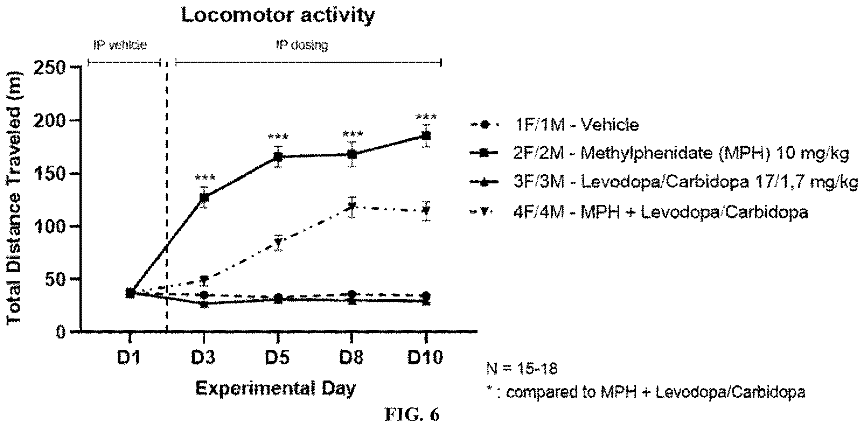

As shown in Figure 6, the total distance travelled was significantly increased after each repeated injection of methylphenidate. Indeed, methylphenidate chronic administration at 10 mg/kg induced a significant increase in mice locomotor activity during development of behavioral sensitization (127,39±38,76 meters at day 3 and 185,70±41,44 meters at day 10) compared to vehicle group (35,38±9,35 meters at day 3 and 34,65±6,41 meters at day 10). No difference was observed for levodopa/carbidopa mice group (26,90± 6,96 meters at day 3 and 29,56±6,84 meters at day 10). In addition, levodopa/carbidopa in combination with methylphenidate significantly reduced mice locomotor activity (48,70±17,61 meters at day 3 and 114,22±33,68 meters at day 10) during development of behavioral sensitization compared to methylphenidate treated group.

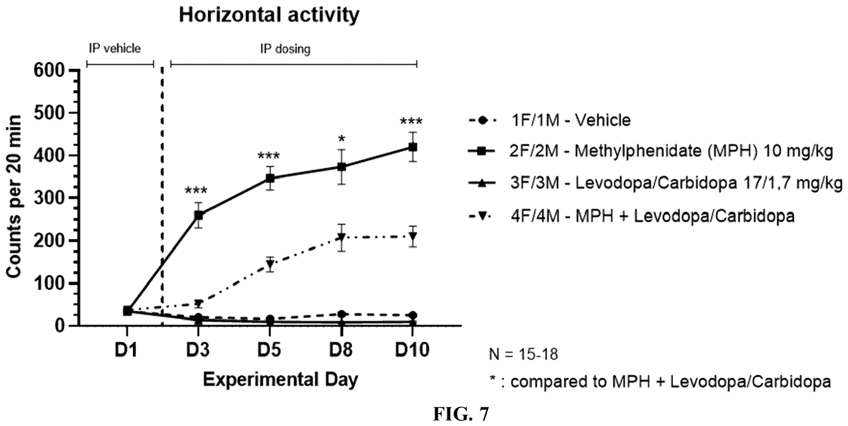

As shown in Figure 7, methylphenidate chronic administration at 10 mg/kg induced a significant increase in mice horizontal activity during development of behavioral sensitization (259,69±115,51 counts at day 3 and 420,38±137,05 counts at day 10) compared to vehicle group (20,44±8,44 counts at day 3 and 24,50±12,39 counts at day 10) and levodopa/carbidopa group (13,72±8,62 counts at day 3 and 9,89±8,63 counts at day 10). In addition, levodopa/carbidopa in combination with methylphenidate significantly reduced mice horizontal activity (51,80±36,21 counts at day 3 and 210,27±94,02 counts at day 10) during development of behavioral sensitization compared to methylphenidate treated group.

This study demonstrates that repeated methylphenidate administrations at 10 mg/kg elicits robust locomotor sensitization and that levodopa/carbidopa reduces motor activity induced by methylphenidate during development of sensitization.

### Effects of levodopa/carbidopa on dependence induced by methylphenidate

Before the beginning of the test, the baseline motor activity was analysed for 10 minutes session.

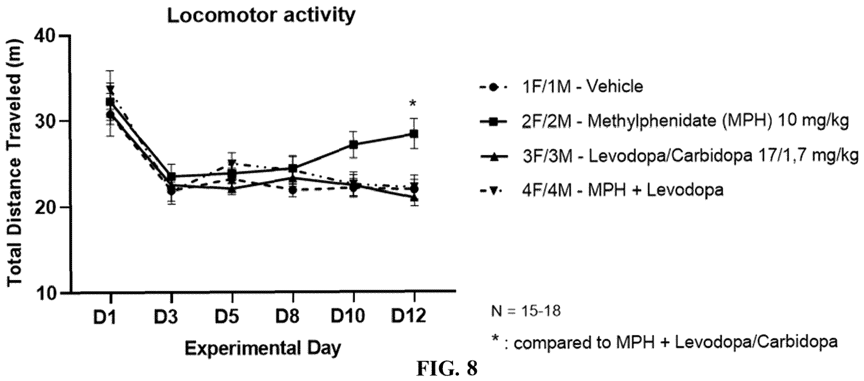

As shown in Figure 8, methylphenidate chronic injection at 10 mg/kg induced a progressive increase in motor activity during baseline sessions. At experimental day 12, a significant increase in mice locomotor activity was observed before treatment administration (28,35±7,43 meters) compared to vehicle group (21,92±4,49 meters). Interestingly, levodopa/carbidopa with methylphenidate significantly reduced mice locomotor activity induced by methylphenidate before treatment injection (22,16±5,47 meters).

In the context of drug addiction, classical conditioning is a prevalent phenomenon. During repeated administration of methylphenidate (or another stimulant), drug-paired environmental stimuli (i.e, the conditioned stimulus) become associated with and potentially predict the availability of the drug (i.e, the unconditioned stimulus). After a learning period, the environmental stimuli alone acquire the ability to produce behavioral activation.

After learning, the only presence of these stimuli triggers behavior adapted to obtaining the reward. That's why, methylphenidate treated mice explore their environment compulsively. These mice anticipate and associate the present situation with situation that will follow. This phenomenon wasn't observed when mice received methylphenidate with levodopa/carbidopa. Thus, this combination has an effect on dependence induced by methylphenidate.

Therefore, it is concluded that administration with 10 mg/kg of methylphenidate (60mg human abuse dose) increases horizontal and locomotor activity of treated mice during development of sensitization and leads to a dependence. Our findings support that levodopa/carbidopa plays an inhibitory role in methylphenidate-induced hyperactivity, development of behavioral sensitization and dependence in male and female mice.

## Claims

1. A composition comprising levodopa for use in preventing the development of addiction and/or dependence to a stimulant in a subject, wherein said subject is treated or is to be treated with the stimulant.

2. The composition for use according to claim 1, wherein said composition prevents the development of sensitization induced by the stimulant in said subject.

3. The composition for use according to claim 1 or claim 2, wherein the composition further comprises an inhibitor of aromatic L-amino acid decarboxylase, preferably wherein said inhibitor of aromatic L-amino acid decarboxylase is carbidopa.

4. The composition for use according to any one of claims 1 to 3, wherein the stimulant is selected from the group comprising or consisting of methylphenidate, amphetamine, dexmethylphenidate, serdexmethylphenidate, methylphenidate derivatives amphetamine, modafinil and combinations thereof, preferably wherein the stimulant is methylphenidate or amphetamine.

5. The composition for use according to any one of claims 1 to 4, wherein the amount of levodopa in the composition is at least about 10 mg, preferably between about 10 mg to about 500 mg, more preferably between about 50 mg to about 100 mg.

6. The composition for use according to any one of claims 3 to 5, wherein the amount of carbidopa in the composition is at least about 1 mg, preferably between about 1 mg to about 50 mg, more preferably between about 5 mg to about 10 mg.

7. The composition for use according to any one of claims 1 to 6, wherein said subject is treated or is to be treated with a dose of stimulants of at least about 1 mg per intake, preferably between about 1 mg to about 200 mg, more preferably between about 10 mg to about 100 mg.

8. The composition for use according to any one of claims 1 to 7, wherein the ratio of stimulant to levodopa is comprised between 30: 100 and 120: 100, or wherein the ratio of stimulant/levodopa/carbidopa is comprised between 30: 100: 10 and 120: 100: 10.

9. The composition for use according to any one of claims 1 to 8, wherein said subject suffers from or is diagnosed with attention deficit hyperactivity disorder (ADHD) or narcolepsy.

10. A pharmaceutical composition comprising levodopa and at least one pharmaceutically acceptable excipient, for use in preventing the development of addiction and/or dependence to a stimulant in a subject, wherein said subject is treated or is to be treated with the stimulant.

11. A composition comprising levodopa and a stimulant, preferably wherein said stimulant is selected from the group comprising or consisting of methylphenidate, amphetamine, dexmethylphenidate, serdexmethylphenidate, methylphenidate derivatives amphetamine, modafinil and combinations thereof, more preferably wherein said stimulant is methylphenidate or amphetamine.

12. The composition according to claim 11, wherein the composition further comprises an inhibitor of aromatic L-amino acid decarboxylase, preferably wherein said inhibitor of aromatic L-amino acid decarboxylase is carbidopa.

13. The composition according to any one of claims 11 to 12, wherein the composition comprises a ratio of stimulant to levodopa comprised between 30: 100 and 120: 100, or a ratio of stimulant/levodopa/carbidopa comprised between 30: 100: 10 and 120: 100: 10.

14. The composition according to any one of claims 11 to 13, for use as a medicament.

15. The composition according to any one of claims 11 to 13, for use in treating ADHD or narcolepsy in a subject in need thereof.
